# FASCICULE DE BREVET EUROPEEN

(11) **EP 1 129 746 B1**
(45) Date de publication et mention de la délivrance du brevet: **04.01.2006**
(21) Numéro de dépôt: 01400501.1
(22) Date de dépôt: 27.02.2001
(51) Int. Cl.: A61N 1/39, A61N 1/362

(54) **Dispositif médical implantable actif de type défibrillateur/cardioverteur implantable à gestion perfectionnée des tachycardies ventriculaires**
Aktives implantierbares medizinisches Gerät von der Art implantierbaren Defibrillators/Kardioverters mit verbesserter Kontrolle von ventrikulärer Tachykardie
Active implantable medical device of the type implantable defibrillator/cardioverter with improved control of ventricles tachycardia

(30) Priorité: 29.02.2000 FR 0002526
(43) Date de publication de la demande: 05.09.2001
(73) Titulaire: ELA MEDICAL, F-92541 Montrouge (FR)
(72) Inventeur: Limousin, Marcel, 75014 Paris (FR)
(74) Mandataire: Dupuis-Latour, Dominique

(56) Documents cités:
- EP-A- 0 862 927
- WO-A-98/40122
- WO-A-98/55178
- US-A- 5 999 850

## Description

L'invention concerne les "dispositifs médicaux implantables actifs" tels que définis par la directive 90/385/CEE du 20 juin 1990 du Conseil des communautés européennes, et plus particulièrement la famille des appareils chargés de délivrer au coeur des impulsions électriques de haute énergie (c'est-à-dire dépassant notablement l'énergie fournie pour la simple stimulation) pour tenter de mettre fin à une tachyarythmie. Ces modes de thérapie incluent également un mode de stimulation programmé à faible énergie et haute fréquence ou "ATP" (*AntiTachycardic Pacing*).

Ces dispositifs sont appelés "défibrillateurs implantables" ou "cardioverteurs implantables", étant entendu que l'invention couvre aussi bien les défibrillateurs/cardioverteurs/stimulateurs ou les défibrillateurs/stimulateurs implantables.

Ces dispositifs comportent un générateur d'impulsions chargé de surveiller l'activité cardiaque et de générer des impulsions de haute énergie quand le coeur présente une arythmie ventriculaire susceptible d'être traitée. Quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation".

Les EP-A-0 626 182 (ELA Médical) et EP-A-0 838 235 (ELA Médical) décrivent de tels dispositifs.

La tachyarythmie recouvre en fait plusieurs situations très différentes, à savoir la fibrillation ventriculaire (FV), la tachycardie ventriculaire (TV), la tachycardie sinusale (TS) et la tachycardie supraventriculaire (TSV, qui est en fait une tachycardie d'origine auriculaire).

Dès qu'un trouble du rythme ventriculaire est reconnu et avéré (tachycardie ventriculaire ou fibrillation ventriculaire), les défibrillateurs actuellement disponibles appliquent généralement une thérapie différenciée :
- en cas de tachycardie ventriculaire organisée, application d'une stimulation antitachycardique (ATP), puis application d'un choc si cette stimulation ATP est inefficace ; et,
- en cas de fibrillation ventriculaire, application immédiate d'un choc, qui est la seule thérapie envisageable car le pronostic vital du patient est alors en jeu.

Dans le premier cas (TV organisée), hormis quelques épisodes syncopaux, l'urgence d'une thérapie par choc est moindre que dans le cas d'une FV, surtout lorsque la tachycardie est de fréquence relativement lente. Certaines études suggèrent en fait que deux populations, l'une traitée d'abord avec ATP, l'autre immédiatement par un choc, présentent en fait durant le suivi un nombre de chocs sensiblement identique. Ceci semblerait suggérer que l'ATP, même si elle est efficace, n'empêche pas la survenue rapide d'un événement plus grave nécessitant un traitement par choc.

L'inconvénient d'un traitement par choc immédiat est cependant la douleur ressentie par le patient, et la situation d'inconfort vécue au quotidien dans la crainte d'un futur choc.

Il est également suggéré par certaines études que de nombreuses TV pourraient se terminer spontanément, s'il était possible d'attendre. Malheureusement il est en général nécessaire d'intervenir rapidement car ces TV peuvent être mal supportées.

Il apparaît donc souhaitable de disposer d'un dispositif permettant, en cas de tachycardie ventriculaire organisée d'appliquer au moins provisoirement une thérapie autre qu'une thérapie de choc, mais plus efficace que la stimulation antitachycardique ATP jusqu'à présent utilisée.

Le WO-A-98/40122 propose de délivrer sur une électrode auxiliaire, placée dans un ventricule, une impulsion de niveau intermédiaire entre impulsion de stimulation et de choc, mieux tolérée par le patient. L'invention propose une autre solution, appliquée à un dispositif médical du type connu d'après le EP-A-0 838 235, c'est-à-dire comprenant : des moyens de délivrance d'une thérapie de défibrillation et/ou de cardioversion et/ou de stimulation antitachycardique ventriculaire ; et des moyens de recueil de l'activité cardiaque ; et des moyens de détection dans l'activité ainsi recueillie d'un trouble du rythme ventriculaire distinct d'une fibrillation ventriculaire.

Selon l'invention, ce dispositif comprend en outre des moyens de stimulation biventriculaire, reliés à au moins deux sites ventriculaires, droit et gauche, et déclenchés à détection dudit trouble du rythme ventriculaire, cette stimulation étant une stimulation synchrone à faible énergie des ventricules droit et gauche. Les sous-revendications visent des mises en oeuvre avantageuses.

On va maintenant exposer plus en détail, à titre d'exemple, la manière dont l'invention peut être mise en oeuvre.

Le dispositif médical de l'invention est un défibrillateur ou cardioverteur d'un type en lui-même connu et du type dit "multisite", c'est-à-dire dans lesquelles des électrodes sont placées en une pluralité de sites respectifs distincts, avec au moins deux sites ventriculaires. Il peut s'agir d'un dispositif de type "double chambre" (double stimulation ventriculaire), "triple chambre" (stimulation atriale droite et double stimulation ventriculaire) ou même "quadruple chambre" (double stimulation atriale et double stimulation ventriculaire).

Essentiellement, l'invention propose d'améliorer la tolérance hémodynamique de certaines tachycardies par une stimulation synchrone des ventricules droit et gauche, soit sur un mode déclenché par la détection dans une chambre, soit par un léger "overdriving" (stimulation conjointe des deux ventricules).

De plus, la resynchronisation des ventricules peut dans certaines circonstances participer au processus d'arrêt spontané de la tachycardie, par interruption d'un foyer de ré-entrée ou par un ré-homogénéisation des périodes réfractaires ventriculaires.

Cette stimulation des ventricules est opérée par application d'impulsions conventionnelles de stimulation, c'est-à-dire d'impulsions de faible énergie (de l'ordre de quelques microjoules) de type multisite (stimulation à droite et à gauche).

Plus précisément, selon l'invention, le dispositif va prendre une action :
- soit dès que le rythme ventriculaire atteint une fréquence seuil, par exemple 120 cpm ;
- soit dès qu'une tachycardie ventriculaire est détectée (par exemple de la manière décrite par les EP-A-0 626 182 et EP-A-0 838 235 précités) ;
- soit, optionnellement, si un ou plusieurs capteurs d'activité indiquent l'absence d'activité et si le rythme ventriculaire demeure supérieur à une fréquence de seuil.

Alors, le dispositif va délivrer une stimulation ventriculaire gauche, synchrone de la détection ventriculaire droite (resynchronisation).

Avantageusement, on peut prévoir une stimulation avec raccourcissement de l'intervalle d'échappement ventriculaire par rapport à une stimulation simple, tout en maintenant la stimulation biventriculaire.

Si la TV ne s'est pas arrêtée spontanément dans un délai acceptable, par exemple après un délai programmable (de 10 à 10 000 cycles ventriculaires par exemple), la thérapie classique d'arrêt de la TV est délivrée par l'appareil, qu'il s'agisse d'une stimulation ATP ou de l'application d'un choc de défibrillation ou de cardioversion.

Dans le cas où une stimulation ATP est délivrée, celle-ci peut être délivrée en monosite ou en multisite.

## Revendications

1. Un dispositif médical implantable actif de type défibrillateur/cardioverteur implantable, comprenant :
- des moyens de délivrance d'une thérapie de défibrillation et/ou de cardioversion et/ou de stimulation antitachycardique ventriculaire,
- des moyens de recueil de l'activité cardiaque, et
- des moyens de détection dans l'activité ainsi recueillie d'un trouble du rythme ventriculaire distinct d'une fibrillation ventriculaire,
dispositif **caractérisé en ce qu'**il comprend en outre :
- des moyens de stimulation biventriculaire, reliés à au moins deux sites ventriculaires, droit et gauche, et déclenchés à détection dudit trouble du rythme ventriculaire, cette stimulation étant une stimulation synchrone à faible énergie ventricules droit et gauche.

2. Le dispositif de la revendication 1, dans lequel les moyens de détection d'un trouble du rythme ventriculaire sont des moyens de détection du dépassement d'un seuil de fréquence du rythme ventriculaire.

3. Le dispositif de la revendication 1, dans lequel les moyens de détection d'un trouble du rythme ventriculaire sont des moyens de détection d'épisodes de tachycardie ventriculaire.

4. Le dispositif de la revendication 1, comprenant en outre :
- un capteur d'activité, et
- des moyens de resynchronisation de la stimulation du ventricule gauche sur l'activité ventriculaire droite détectée, ces moyens étant déclenchés lorsque le capteur indique une absence d'activité et que le rythme ventriculaire est supérieur à un seuil de fréquence prédéterminé.

5. Le dispositif de la revendication 1, dans lequel, lorsqu'ils sont déclenchés, les moyens de stimulation biventriculaire opèrent avec réduction de l'intervalle d'échappement ventriculaire.

6. Le dispositif de la revendication 1, dans lequel les moyens de détection d'un trouble du rythme ventriculaire sont aptes, en cas de détection persistante, à déclencher les moyens de délivrance de la thérapie de défibrillation et/ou de cardioversion et/ou de stimulation antitachycardique ventriculaire.

7. Le dispositif de la revendication 6, dans lequel les moyens de délivrance de la thérapie de défibrillation et/ou de cardioversion et/ou de stimulation antitachycardique ventriculaire sont déclenchés après écoulement d'un délai prédéterminé de détection continue d'un trouble du rythme ventriculaire.

## Patentansprüche

1. Aktive implantierbare medizinische Vorrichtung vom Typ imlantierbarer Defibrillator/Kardioverter, umfassend:
- Mittel zur Lieferung einer Therapie der Defibrillation und/oder der Kardioversion und/oder der ventrikulären Antitachykardie-Stimulation,
- Mittel zum Empfang der Herzaktivität, und
- Mittel zur Ermittlung, in der so empfangenen Aktivität, einer Störung des ventrikulären Rhythmus, die von einer ventrikulären Fibrillation verschieden ist,
wobei die Vorrichtung **dadurch gekennzeichnet ist, dass** sie darüber hinaus umfasst:
- Mittel zur biventrikulären Stimulation, die mit mindestens zwei ventrikulären Orten, rechts und links, verbunden sind, und bei der Ermittlung der Störung des ventrikulären Rhythmus ausgelöst werden, wobei diese Stimulation eine synchrone Stimulation von schwacher Energie des rechten und des linken Ventrikels ist.

2. Vorrichtung gemäß Anspruch 1, in der die Mittel zur Ermittlung einer Störung des ventrikulären Rhythmus Mittel zur Ermittlung des Überschreitens einer Frequenzschwelle des ventrikulären Rhythmus sind.

3. Vorrichtung gemäß Anspruch 1, in der die Mittel zur Ermittlung einer Störung des ventrikulären Rhythmus Mittel zur Ermittlung von Episoden ventrikulärer Tachykardie sind.

4. Vorrichtung gemäß Anspruch 1, umfassend darüber hinaus:
- einen Aktivitätssensor und
- Mittel zur Resynchronisierung der Stimulation des linken Ventrikels mit der ermittelten Aktivität des rechten Ventrikels, wobei diese Mittel ausgelöst werden, wenn der Sensor eine Abwesenheit der Aktivität anzeigt und wenn der ventrikuläre Rhythmus höher als ein vorbestimmter Frequenzschwellenwert ist.

5. Vorrichtung gemäß Anspruch 1, in der die Mittel zur biventrikulären Stimulation, wenn sie ausgelöst werden, mit einer Verringerung des ventrikulären Auslassintervalls arbeiten.

6. Vorrichtung gemäß Anspruch 1, in der die Mittel zur Ermittlung einer Störung des ventrikulären Rhythmus dazu angepasst sind, im Fall einer anhaltenden Ermittlung die Mittel zur Lieferung der Therapie der Defibrillation und/oder Kardioversion und/oder ventrikulären Antitachykardie-Stimulation auslösen.

7. Vorrichtung gemäß Anspruch 6, in der die Mittel zur Lieferung der Therapie der Defibrillation und/oder Kardioversion und/oder ventrikulären Antitachykardie-Stimulation nach dem Verstreichen einer vorbestimmten Dauer der kontinuierlichen Ermittlung einer Störung des ventrikulären Rhythmus ausgelöst werden.

## Claims

1. An active implantable medical device of the implantable defibrillator/cardiovertor type, including:
- means for delivering a defibrillation and/or cardioversion and/or ventricular antitachycardic stimulation therapy mode,
- means for sensing cardiac activity, and
- means for detecting in the thus sensed activity a disorder of the ventricular rhythm that is distinct from a ventricular fibrillation,
said device being **characterised in that** it further comprises:
- means for biventricular stimulation, connected to at least two ventricular, right and left, sites, and triggered upon detection of said ventricular rhythm disorder, said stimulation being a low energy synchronous stimulation of the right and left ventricles.

2. The device of claim 1, wherein the means for detecting a ventricular rhythm disorder is a means for detecting the crossing of a frequency threshold of the ventricular rhythm.

3. The device of claim 1, wherein the means for detecting a ventricular rhythm disorder is a means for detecting episodes of ventricular tachycar dia.

4. The device of claim 1, further comprising:
- an activity sensor, and
- means for resynchronising the stimulation of the left ventricle on the detected right ventricular activity, said means being triggered when the activity sensor indicates an absence of activity and the ventricular rhythm is higher than a predetermined frequency threshold.

5. The device of claim 1, wherein, when triggered, the means for biventricular stimulation operates with a reduction of the ventricular escape interval.

6. The device of claim 1, wherein the means for detecting a ventricular rhythm disorder is adapted, in case of persistent detection, to trigger the means for delivering the defibrillation and/or cardioversion and/or ventricular antitachycardic stimulation therapy.

7. The device of claim 6, wherein the means for delivering the defibrillation and/or cardioversion and/or ventricular antitachycardic stimulation therapy is triggered after a predetermined time period of continuous detection of a ventricular rhythm disorder has elapsed.
